# EUROPEAN PATENT SPECIFICATION

(11) **EP 0 308 994 B1**
(45) Date of publication and mention of the grant of the patent: **23.03.1994**
(21) Application number: 88116753.0
(22) Date of filing: 15.06.1984
(51) Int. Cl.: G01N 33/49

(54) **Method of pumping blood**
Verfahren zur Blutabnahme
Méthode pour pomper du sang

(30) Priority: 15.06.1983 GB 8316332
(43) Date of publication of application: 29.03.1989
(62) Divisional of application: 84304055.1
(73) Proprietor: GOROG, Peter, Dulwich Park London SE19 1LS (GB)
(72) Inventor: GOROG, Peter, Dulwich Park London SE19 1LS (GB)
(74) Representative: Raynor, John

(56) References cited:
- EP-A- 0 046 345
- FR-A- 2 205 953
- GB-A- 1 283 787
- US-A- 3 768 496

## Description

This invention relates generally to methods and apparatus for measuring haemostasis, that is to say cessation of bleeding, and to certain other methods and apparatus useful in connection with the determination of haemostasis.

There is an urgent need to provide a simple technique for detecting haemorrhagic disorders, in particular so that the risk of bleeding after surgery can be assessed. At present, a number of different time-consuming tests must be performed in a specialised haematological diagnostic laboratory, to assess the risk of bleeding in a particular patient. Not only is the known procedure time-consuming, and thus unsuitable for use in emergency, but also the results are not always consistent.

A method of in vitro analysis of haemostasis time was suggested by Blakley et al (J. Lab. Clin. Med 89 p1306-1314 (1976). In this method, a roller pump is made to circulate blood through a plastic tube, in which has been provided a hole drilled to simulate damage to a blood vessel. I have found that far more consistent results can be obtained by causing blood to pass through a tube, and piercing the tube whilst the blood flows therein, using a needle having a substantially parallel-sided shank.

European Patent Application No. 0129425, from which the present application is divided, describes and claims apparatus for monitoring the haemostasic reaction of a blood sample, comprising a container for the blood sample, means for causing the blood sample to flow through a tube, for example a capillary tube, a support for the tube, and a needle having a substantially parallel-sided shank mounted for movement to pierce a hole in a wall, and preferably in both walls of the tube, substantially perpendicular to its surface, and means for monitoring the blood flow through the hole.

The use of such apparatus makes it possible to pierce the hole in the tube whilst the blood is actually flowing therein, and it has been found that this provides much more consistent results.

A further and particularly serious defect of the haemostasis measuring techniqe suggested by Blakley et al is that the pump used causes severe and unquantifiable damage to the blood corpuscles, which tends to render the results obtained irrelevant to the in vivo situation or non-reproducible. Any pumping mechanism such as the roller-type peristaltic pump used by Blakley et al will cause physical damage to the blood cells, and thus the likelihood of inconsistent results. Even if physical damage to blood cells cannot be observed, any agitation of this kind is likely to cause the release of ADP, which has an effect on haemostasis.

The susceptibility of blood cells to damage and ADP release is of particular concern in apparatus of this kind, since blood is also subject to settling of the corpuscles therein. Clearly, settling will also have an effect on haemostasis time, and the blood must therefore be kept constantly stirred before it is passed to the measuring tube of the apparatus.

I have now discovered that blood can be pumped, that is to say caused to flow in a tube, and simultaneously stirred to prevent settling, by displacement with a fluid which is immiscible therewith, and which will not cause an adverse reaction with the blood.

Accordingly the invention therefore, there is provided a method for simultaneously stirring a blood sample and causing the sample to flow in a tube which method comprises providing a columnar container for the liquid sample having an outlet for the blood at the lower end thereof in communication with the tube, and causing a displacing fluid to pass into the lower end of the columnar container to stir the blood to prevent sedimentation thereof and simultaneously to displace the blood through the outlet, wherein the displacing fluid is a fluid which is immiscible with and less dense than the blood, and which does not cause substantial damage to the blood.

The displacing fluid is preferably a liquid hydrocarbon such as paraffin oil, and can be pumped into the columnar container by any suitable means, for example using a syringe pump.

The diameter of the columnar container is preferably such that the droplets of the immiscible displacing fluid which form in the column are substantial in size in comparison with the column diameter, and thereby have a substantial stirring effect on all the bulk of liquid in the column.

A preferred embodiment of the invention will now be illustrated in detail with reference to the accompanying drawings, in which:

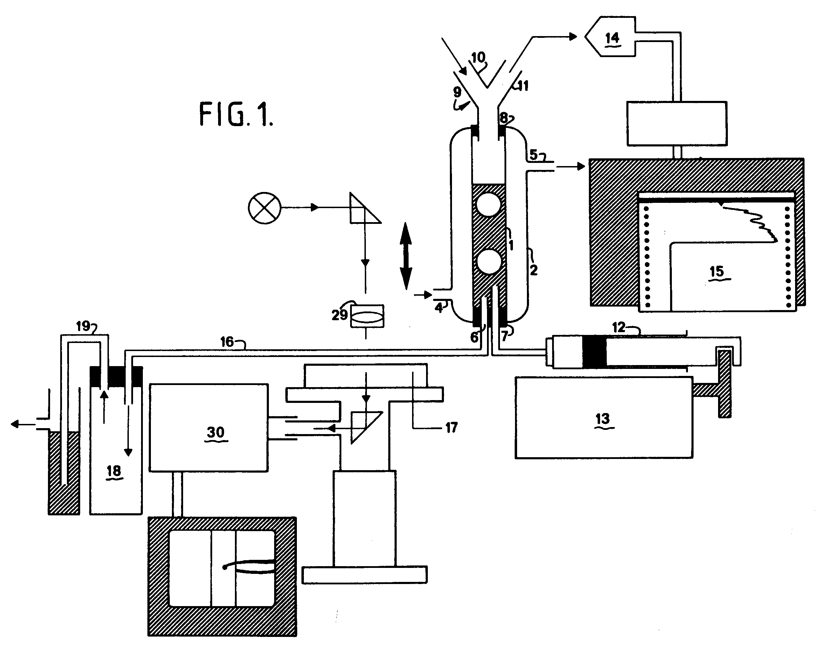

Figure 1 is a schematic diagram of apparatus for measuring the rate of haemostasis in a blood sample, according to EP 0129425 with which the method of the present invention is utilised.

Referring to Figure 1, apparatus for measuring blood haemostasis comprises a container 1 for the blood provided with a water jacket 2 having a water inlet 4 and a water outlet 5. Water at a constant temperature of 37°C may be passed through the waterjacket. Again, this assists consistency of the results measured. The container 1 has an outlet 6 for blood at its lower end, in the form of a tube received in a rubber bung. The bung also receives an inlet 7 for a displacing fluid, for example paraffin oil. Received in a further bung 8 in the upper end of the container 1 is a Y-tube, having arms 10 and 11.

Arm 10 is used for introducing the blood sample into the container 1. After the blood sample is introduced, an amount of the displacing fluid, paraffin oil, is also introduced, to form a layer on the surface of the blood. The arm 10 is then sealed.

Arm 11 is sealed to the atmosphere, and contains a pressure transducer to enable measurement of the blood flow to be recorded.

In an alternative embodiment, the blood outlet 6 and the fluid inlet 7 may be concentric. The inlet 7 is connected to a conventional syringe pump 12, which may be driven by a conventional motor 13.

The pressure transducer 14 in arm 11 of the Y-tube 9 may be connected to conventional data capture apparatus, which may, for example include a chart recorder 15.

A tube 16 which is of polyethylene which could alternatively be made of some other inert plastics material such as polytetrafluoroethylene has an external diameter of 1.00 mm and an internal diameter of 0.5 mm, is connected to outlet 6 from the blood container 1, and passes through a jig 17 and piercing arrangement, which are described in more detail in EP 0129425.

After passage through the jig 17, waste blood is passed into a closed drainage vessel 18. Vessel 18 has a tube 19 connected to its outlet which dips into a pool of mercury. Adjustment of the height of the mercury column in the tube 19 thus provides a readily measurable and variable back-pressure to the blood in the tube 16.

In use of the apparatus, the syringe 12 is filled with light paraffin oil, and the container 1 with anticoagulated blood (for example buffered with heparin or citrate). A new tube 16 is applied to the outlet 6 of tube 1, and cause to pass through jig 17, and into vessel 18. A layer of hydrocarbon oil is floated on the surface of blood, using branch 10 of the Y tube 9, and branch 10 is then sealed.

Operation of the syringe pump 13 is then commenced, until a steady flow of stirred blood is obtained in tube 16. The back-pressure is adjusted to 60 mm Hg, by varying the height of the mercury column in tube 19. The pressure in the blood vessel 1 is meanwhile monitored by means of pressure transducer 14, and the blood is maintained at a constant 37°C by means of waterjacket 2. When the pressure reading on chart recorder 15 indicates that a steady state has been reached, needle 28 is passed through the tube 16, and into a hole, so as to begin the "bleeding". The build-up of a platelet "plug" is meanwhile observed by tracing the pressure rise, using the microscope and television system. Any water flowing through the tube 19 may be sucked off from the mercury surface by suction apparatus.

The pumping technique utilising displacement by hydrocarbon oil has been found to cause no appreciable damage to the red cells. Previous techniques have been found to cause considerable damage, and I have even found that a number of other fluids which might have been expected to be inert and therefore as effective as hydrocarbon oil in fact cause considerable damage. For example, silicone fluid has been found to cause some damage to red cells.

The technique described above is suitable for determination of the haemostasis reaction of non-anticoagulated blood that is to say, blood to which no anti-coagulant has been added, provided that the blood sample is used immediately after being taken. The technique indicates that haemostasis is generally complete in from 3 to 5 minutes.

When haemostasis is complete, a steady perfusion pressure baseline is obtained until the blood clots. That moment is sharply indicated by the sudden increase of the pressure.

Thus, information regarding both the haemostatic activity and the coagulation of the blood can be obtained from the same, non-anticoagulated blood sample.

It will of course be readily understood that a large number of variations of the above-described specific embodiment are possible, within the scope of the present invention.

## Claims

1. A method for simultaneously stirring a blood sample and causing the sample to flow in a tube (16) which method comprises providing a columnar container (1) for the liquid sample having an outlet (6) for the blood at the lower end thereof in communication with the tube (16), and causing a displacing fluid, to pass into the lower end of the columnar container (1) to stir the blood to prevent sedimentation thereof and simultaneously to displace the blood through the outlet (6), wherein the displacing fluid is a fluid which is immiscible with and less dense than the blood, and which does not cause substantial damage to the blood.

2. A method as claimed in Claim 1, wherein the columnar container (1) is such that droplets of the displacing fluid formed in the column are substantial in size in comparision with the diameter of the columnar container (1).

3. A method as claimed in Claim 1 or Claim 2, wherein the displacing fluid is a hydrocarbon oil.

4. A method as claimed in any one of the preceding claims, including means (2) are provided for maintaining the blood sample at a substantially constant temperature.

## Patentansprüche

1. Verfahren zum gleichzeitigen Rühren einer Blutprobe und Veranlassen, daß die Probe in ein Rohr (16) strömt, wobei das Verfahren aufweist das Vorsehen eines säulenförmigen Behälters (1) für die Flüssigprobe mit einem Auslaß (6) für das Blut an seinem unteren Ende in Verbindung mit dem Rohr (16), und das Veranlassen, daß ein Verdrängungsfluid in das untere Ende des säulenförmigen Behälters (1) zum Rühren des Blutes geht, um dessen Sedimentation zu verhindern, und gleichzeitig das Blut durch den Auslaß (6) verdrängt, wobei das Verdrängungsfluid ein Fluid ist, welches mit dem Blut nicht vermischbar ist und weniger dicht ist als das Blut und welches keine wesentliche Beschädigung an dem Blut verursacht.

2. Verfahren nach Anspruch 1, wobei der säulenförmige Behälter (1) derart ist, daß Tröpfchen des Verdrängungsfluides, ausgebildet in der Säule, im wesentlichen eine Größe vergleichbar mit dem Durchmesser des säulenförmigen Behälters (1) haben.

3. Verfahren nach Anspruch 1 oder Anspruch 2, wobei das Verdrängungsfluid ein Kohlenwasserstoff-Öl ist.

4. Verfahren nach einem der vorhergehenden Ansprüche, mit einer Einrichtung (2), welche vorgesehen wird zum Halten der Blutprobe auf einer im wesentlichen konstanten Temperatur.

## Revendications

1. Procédé pour agiter un échantillon de sang et, en même temps, pour le faire couler dans un tube (16), ce procédé consistant à utiliser, pour l'échantillon liquide, un récipient en forme de colonne (1) qui comporte, à son extrémité inférieure, un orifice de sortie (6) pour le sang en communication avec le tube (16), et à envoyer un liquide de déplacement à l'extrémité inférieure du récipient en forme de colonne (1) pour agiter le sang afin d'empêcher sa sédimentation et, en même temps, pour déplacer le sang à travers l'orifice de sortie (6), le liquide de déplacement étant un liquide qui est non miscible au sang et moins dense que celui-ci et qui ne produit pas d'altération appréciable du sang.

2. Procédé selon la revendication 1, dans lequel le récipient en forme de colonne (1) est tel que des gouttelettes du liquide de déplacement formées dans la colonne aient une dimension substantielle par rapport au diamètre du récipient en forme de colonne (1).

3. Procédé selon la revendication 1 ou 2, dans lequel le liquide de déplacement est une huile hydrocarbonée.

4. Procédé selon l'une quelconque des revendications 1 à 3, dans lequel des moyens (2) sont prévus pour maintenir l'échantillon de sang à une température pratiquement constante.
